# EUROPEAN PATENT APPLICATION

(11) **EP 2 545 960 A1**
(43) Date of publication of application: **16.01.2013**
(21) Application number: 12075086.4
(22) Date of filing: 13.07.2012
(51) Int. Cl.: A61N 1/36, A61F 11/04, H04R 25/00

(54) **Fully-implantable, microphoneless cochlear implant**

(30) Priority: 13.07.2011 US 201161507320 P; 21.06.2012 US 201213529011
(71) Applicant: Envoy Medical Corporation, St. Paul, MN 55110 (US)
(72) Inventor: Beckerle, Travis M., St. Paul, Minnesota 55104 (US); Halfen, Joseph J., Woodbury, Minnesota 55129 (US); Glumac, Daniel E., Lino lakes, Minnesota 55038 (US); Mazanec, Paul R., Blaine, Minnesota 55449 (US); Nelson, Daniel J., Ramsey, Minnesota 55303 (US); Schiller, Peter J., Coon Rapids, Minnesota 55443 (US); Verzal, Kevin E., Lino Lakes, Minnesota 55014 (US); Wallenfelt, Brian P., Plymoth, Minnesota 55442 (US)
(74) Representative: Pfenning, Meinig & Partner GbR

(57) **Abstract**

A fully implantable apparatus for improving the hearing of a hearing-impaired subject is shown, said apparatus comprising a means for sensing vibrations impinging upon the tympanic membrane or an object in operative contact with the tympanic membrane, a means for converting said vibrations to an electrical signal, and a means for transmitting the electrical signal to the inner ear.

## Description

### FIELD OF THE INVENTION

The present application relates generally to cochlear implants and methods of improving the hearing of a subject using such implants. More particularly, the application relates to cochlear implants that are fully-implantable and microphoneless and methods of improving the hearing of a subject using such fully-implantable, microphoneless implants.

### BACKGROUND OF THE INVENTION

In an anatomically normal human hearing apparatus, sound waves, which represent acoustical energy, are directed into an ear canal by the outer ear (pinna) and impinge upon a tympanic membrane (eardrum) interposed, at the terminus of the ear canal, between the ear canal and the middle ear space. The pressure of the sound waves effect tympanic vibrations in the eardrum, which then become manifested as mechanical energy. The mechanical energy in the form of tympanic vibrations is communicated to the inner ear by a sequence of articulating bones located in the middle ear space, which are generally referred to as the ossicular chain. The ossicular chain must be intact if acoustical energy existing at the eardrum is to be conducted as mechanical energy to the inner ear.

The ossicular chain includes three primary components, the malleus, the incus, and the stapes. The malleus includes respective manubrium, neck, and head portions. The manubrium of the malleus attaches to the tympanic membrane at a point known as the umbo. The head of the malleus, connected to the manubrium by the neck portion, articulates with one end of the incus, which provides a transmission path for the mechanical energy of induced vibrations from the malleus to the stapes. The stapes includes a capitulum portion connected to a footplate portion by means of a support crus and is disposed in and against a membrane-covered opening to the inner ear, referred to as the oval window. The incus articulates the capitulum of the stapes to complete the mechanical transmission path.

Normally, tympanic vibrations are mechanically conducted through the malleus, incus, and stapes, to the oval window and through to the inner ear (cochlea). These mechanical vibrations generate fluidic motion (transmitted as hydraulic energy) within the cochlea. Pressures generated in the cochlea by fluidic motion are accommodated by a second membrane-covered opening between the inner and middle ear, referred to as the round window. The cochlea translates the fluidic motion into neural impulses corresponding to sound perception as interpreted by the brain. However, various disorders of the tympanic membrane, ossicular chain and/or inner ear can occur to disrupt or impair normal hearing.

Hearing loss, which may be due to many different causes, is generally of two types, conductive and sensorineural. Of these types, conductive hearing loss occurs where the normal mechanical pathways for sound to reach the hair cells in the cochlea are impeded, for example, by damage to the ossicles. Conductive hearing loss may often be helped by use of conventional hearing aids, which amplify sound so that acoustic information does reach the cochlea and the hair cells. Other times, conductive hearing loss can be helped by the use of a middle ear implant, which essentially augments or bypasses the mechanical conduction of the ossicular chain. Some examples of such a middle ear implant can be found in U.S. Patent Nos. 4,729,366 and 4,850,962 to Schaefer.

In many people who are profoundly deaf, however, the reason for deafness is sensorineural hearing loss. This type of hearing loss is due to the absence of, or destruction of, the hair cells in the cochlea which transduce acoustic signals into nerve impulses. These people are thus unable to derive suitable benefit from conventional hearing aid or middle ear implant systems, because there is damage to or absence of the mechanism for nerve impulses to be generated from sound in the normal manner.

It is for the purpose of helping these profoundly deaf people that cochlear implant systems have been developed. Such systems bypass the hair cells in the cochlea and directly deliver electrical stimulation to the auditory nerve fibers, thereby allowing the brain to perceive a hearing sensation resembling the natural hearing sensation normally delivered to the auditory nerve. U.S. 4,532,930 provides a description of one type of traditional cochlear implant system.

A typical system includes an external microphone, signal processor and transmitter, and an implanted receiver and electrode. The microphone transponds normal sound waves, converting this acoustic or mechanical sound energy into electrical energy representative thereof. The processor amplifies the electrical energy, filters it and sends it to the transmitter, which changes the electrical signals into magnetic signals. Transcutaneous magnetic currents cross the skin and are received by the implanted receiver, a coil for example, and the signal travels to the cochlea via a wire electrode. Current flows between this active electrode and a nearby ground electrode, preferably disposed in the Eustachian tube, to stimulate nerve fibers present in the cochlea. The brain interprets this stimulation as sound. See T. Kriewall, "Why Combine Multichannel Processing With a Single Electrode," Hearing Instruments, June 1985; W. House, D. Bode, and K. Berliner, "The Cochlear Implant: Performance of Deaf Patients," Hearing Instruments, September 1981.

The implanted stimulator/receiver has typically included the antenna receiver coil that receives the coded signal and power from the external processor component and a stimulator that processes the coded signal and outputs a stimulation signal to an intracochlea electrode assembly, which applies the electrical stimulation directly to the auditory nerve producing a hearing sensation corresponding to the original detected sound. As such, the implanted stimulator/receiver device has been a relatively passive unit that has relied on the reception of both power and data from the external unit to perform its required function. The external componentry of the cochlear implant has been traditionally carried on the body of the subject, such as in a pocket of the subject's clothing, a belt pouch, or in a harness, while the microphone has been mounted on a clip mounted behind the ear or on a clothing lapel of the subject. As such, traditional systems have required a large amount of external componentry and electrical leads to enable the system to function properly.

More recently, due in the main to improvements in technology, the physical dimensions of the speech processor have been reduced, thereby allowing the external componentry to be housed in a small unit capable of being worn behind the ear of the subject. This unit has allowed the microphone, power unit, and the speech processor to be housed in a single unit capable of being discreetly worn behind the ear. Despite the development of these behind-the-ear (BTE) units, there still exists the need for an external transmitter coil to be positioned on the side of the subject's head to allow for the transmission of the coded sound signal from the speech processor and power to the implanted stimulator unit. This need for a transmitter coil further requires leads and additional componentry, which have added to the complexity and conspicuousness of such systems. Nevertheless, the introduction of a combined unit capable of being worn behind the ear has greatly improved the visual and aesthetic aspects for cochlear implant subjects.

While traditional cochlear implants have proven very successful in restoring hearing sensation to-many people, the construction of the conventional implant with its external electronic components has limited the circumstances in which the implant can be used by a subject. For example, subjects cannot wear the devices while showering or engaging in water-related activities. Most implantees also do not use the devices while asleep due to discomfort caused by the presence of the BTE unit. With the increasing desire of cochlear implant implantees to lead a relatively normal life, there exists a need to provide a system which allows for total freedom with improved simplicity and reliability.

Because of this need, fully implantable systems that do not require external componentry for operation for at least some of their operating life, have been postulated, although none have as yet been commercially available.

An example of one type of system which has been proposed is described in U.S. Pat. No. 6,067,474 by Advanced Bionics Corporation and Alfred E. Mann Foundation for Scientific Research. This system attempts to provide all system components implanted in the subject and includes a microphone placed in the ear canal which communicates with a conventionally positioned receiver unit via a conventional RF link. There is further described a battery unit which can be integrated with the receiver unit or separate therefrom. Such a system provides further complications as it requires surgical implantation of a number of components and hence complicates the surgical procedure. The system also maintains the need for a RF link during normal operation between implanted components, which increases overall power requirements of the system, and thus often rapidly drains the internal battery supply.

Another proposed device is described in International Patent Application No. WO 01/39830 to EPIC Biosonics Inc. This system also employs a microphone positioned in the ear canal and an extendible lead connecting the microphone to the implanted stimulator. This proposed system therefore inherits many of the drawbacks of the system mentioned above.

Also, both of these proposed implants rely on the use of a microphone. Microphones do not provide sound quality on par with that of sound measured as it is naturally amplified by the pinna and ear canal, or as it is sensed by the ossicular chain. Also, all of the above-mentioned implants would require full explantation if any component needed updating or replacement.

With the above background in mind, there is a need to provide a totally implanted cochlear implant that does not require external componentry to operate at least for a specific period of time. Preferably, the implant will use sound input as received by the ossicular chain, whether that chain is intact or not, rather than relying on a microphone. Also preferably, the implant will allow some components, namely the processor, to be easily replaced without requiring full explantation.

Any discussion of documents, acts, materials, devices, articles or the like which has been included in the present specification is solely for the purpose of providing a context for the present invention. It is not to be taken as an admission that any or all of these matters form part of the prior art or were common general knowledge in the field relevant to the present invention as it existed before the priority date of each claim of this application.

### SUMMARY OF THE INVENTION

The present invention is directed toward a fully-implantable cochlear implant and method for improving the hearing of a subject using such a fully-implantable cochlear implant. In accordance with one aspect of the present invention the mechanical vibrations that would, if a subject is anatomically normal, impinge on the tympanic membrane and subsequently be transmitted to the ossicular chain of the middle ear are sensed by a sensor in operative contact with the tympanic membrane or one or more of the bones of the middle ear. The sensor can convert the mechanical vibrations into electrical signals, which signals are then interpreted and manipulated by a processor. The processor then sends an output electrical signal to the cochlea via a cochlear electrode where the output electrical signals are ultimately interpreted by the subject as auditory information. This implant is an improvement over the prior art because it is microphoneless. The auditory information of sounds picked up by a microphone has lower quality than sounds that have traveled through the subject's ear canal. Directionality and amplification of useful frequencies are generally improved in sounds that have traveled through the ear canal. Thus, the inventive implant will provide superior results compared to those of other currently available or proposed cochlear implants.

A further advantage of the current invention is that the ossicular chain of the middle ear may not have to be disarticulated. Disarticulation is usually required when sensing mechanical vibrations at the tympanic membrane or ossicular chain because of feedback issues. The present implant, however, does not rely on transmitting the sound information through mechanical vibrations. Instead it relies on electrical stimulation of the cochlea and, thus, as long as the sensing portion of the implant is electrically isolated (vs. mechanically isolated), there should be no feedback or interference issues. Being able to leave the ossicular chain intact is an advantage because it simplifies the surgical procedure.

To ensure the cochlear implant device is fully-implantable, the present invention also contemplates a long-lasting, rechargeable battery. The battery is chargeable by telemetry and, as a result, an inductive coil is part of the fully-implantable cochlear implant. A separate battery charger for the implanted battery can also be envisaged. This separate charger can charge the implanted battery through use of the inductive link provided by internal and external coils. A separate charger allows the implanted power source to be recharged when required.

In one embodiment of the invention, at least the cochlear electrode is attached to the processor with a removable lead, whereby if for some reason, the processor needed to be replaced or updated, the cochlear electrode could remain in position inside the subject. Being able to leave the electrode in place is advantageous because the cochlear tissue is fragile and can be damaged by insertion or removal of an electrode. Having the cochlear tissue be as intact as possible is important to retain any natural hearing function the subject may have and to allow for the cochlea to function as needed to stimulate the auditory nerve, which a subject will interpret as auditory information. Also, a further advantage of a removable lead is that the cochlea may ossify after implantation making replacement difficult or outright impossible. The invention also contemplates that all the implanted electrodes of the device may be attached to the processor by removable leads, conferring the same advantage of being able to easily remove the processor without having to disturb any other portions of the fully-implantable cochlear implant. In addition, this invention contemplates that the processor may be attached to a sensor that has been previously implanted for use with another hearing restoration system, such as the ESTEEM™ implant (Envoy Medical Corporation, St. Paul, MN). This is advantageous because the subject can have a cochlear implant implanted without replacing the sensor if the subject's hearing changes due to, for example, progressive hearing loss. Being able to leave the sensor in-place is advantageous because the surgical procedure to properly place the sensor may be challenging and time consuming.

In another embodiment of the invention, the cochlear electrode is marked with a radioactive compound. Such marking is advantageous because it allows for ease in determining the correct placement of the electrode during implantation procedures. It is also advantageous because it allows for ease in determining if the electrode has moved out of position after implantation is complete, for example, when trying to diagnose why an implant no longer functions as intended.

In still another embodiment of the invention, the cochlear electrode is relatively short and does not require navigating the turns of the cochlea conch shape. Being able to avoid navigating such turns is advantageous because it reduces the risk of damaging the tissue of the cochlea.

### BRIEF DESCRIPTION OF THE DRAWINGS

The above, as well as other advantages of the present invention, will become readily apparent to those skilled in the art from the following detailed description when considered in the light of the accompanying drawings in which:
FIG. 1 is a schematic coronal section through a portion of the skull of a human subject adjacent to the ear showing the disposition of one embodiment of a fully-implantable, microphoneless cochlear implant in accordance with the present invention.
FIG. 2 is a schematic of the fully-implantable, microphoneless cochlear implant.
FIGS. 3A and 3B are schematics showing the processor of the fully-implantable cochlear implant and a removable lead. FIG. 3A shows the processor of the fully-implantable cochlear implant with the lead removed. FIG. 3B shows the processor of the fully-implantable cochlear implant with the lead attached.
FIG.4 is a schematic of the sensor of the fully-implantable cochlear implant touching the malleus bone of the middle ear.
FIG. 5 is a schematic of the middle ear of a subject with the sensor of the fully-implantable cochlear implant touching the incus bone of the middle ear.
FIGS. 6A and 6B are schematics of the processor of the fully-implantable cochlear implant. FIG. 6A shows an embodiment of the invention where three removable leads attach to the electronics unit of the processor. FIG. 6B shows an embodiment of the invention where two removable leads attach to the electronics unit of the processor.
Fig. 6C is a more detailed view of the processor illustrated in Fig. 6B.
FIGS. 7A and 7B are schematics showing the end of a removable lead distal to the attachment point to the processor, disposed in which is both a ground electrode and a cochlear electrode as realized in an embodiment of the fully-implantable cochlear implant in accordance with the present invention. FIG. 7A is a close-up schematic showing the two electrodes inside the lead. FIG. 7B shows a potential placement of the two electrodes in relation to a subject's anatomy.
FIG. 8 is a schematic as in Fig. 1, but shows a shorter cochlear electrode as realized in an embodiment of the fully-implantable cochlear implant in accordance with the present invention.
FIG. 9 is a schematic view showing an embodiment of the invention having a non-contact sensor.

### DETAILED DESCRIPTION OF THE PREFERRED EMBODIMENTS

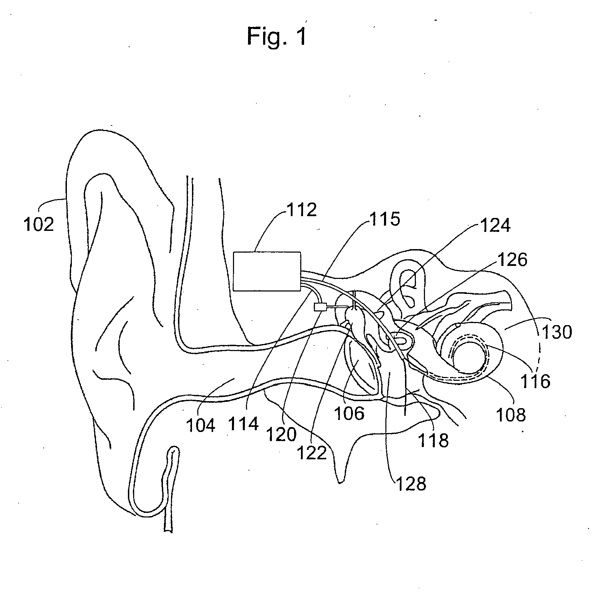

Referring to Fig. 1, an embodiment of a fully-implantable cochlear implant device in accordance with the present invention is shown. The subject, if anatomically normal, possesses at least a pinna 102, an ear canal 104, a tympanic membrane 106, an ossicular chain composed of a malleus 122, an incus 124, and a stapes 126 disposed within a middle ear space 128, and an inner ear 130 including a cochlea 108.

The implant in this embodiment is composed of: a processor 112, a first lead 114 connecting the sensor 120 to the processor 112, a sensor 120, shown here touching the malleus 122 (but could also touch the tympanic membrane 106, the incus 124, or the stapes 126), and a combination lead 115 attached to the processor 112, wherein combination lead 115 contains both a ground electrode 118 and a cochlear electrode 116.

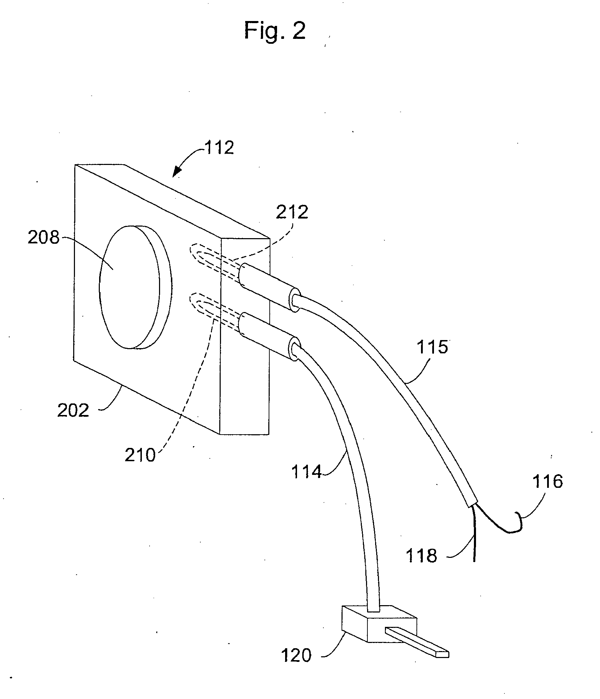

Referring to Fig. 2, an embodiment of a fully-implantable cochlear implant in accordance with the present invention is shown. The device in this embodiment is composed of: a processor 112, a sensor 120, a first lead 114 connecting the sensor 120 to the processor 112, and a combination lead 115 attached to the processor 112, wherein combination lead 115 contains both a ground electrode 118 and a cochlear electrode 116. The processor 112 is itself composed of at least a housing 202, a coil 208, first female receptacle 210 and second female receptacle 212 for insertion of the leads 114 and 115, respectively.

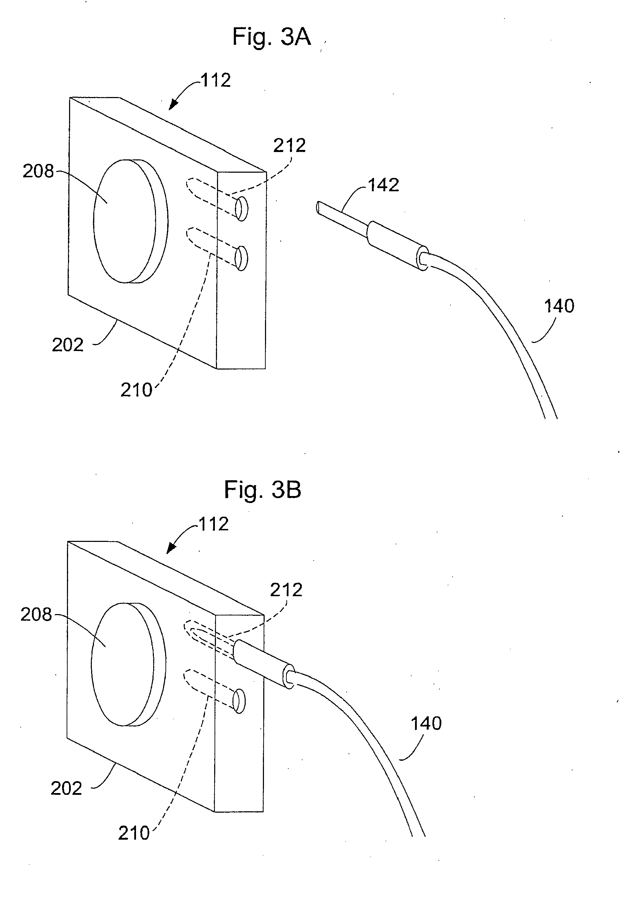

Referring to Fig. 3, the processor 112, at least composed of a housing 202, a coil 208, and a generic lead 140 are shown. The lead 140 is removable and can be attached to the processor 112 by insertion of a male connector 142 of the generic lead 140 into any available female receptacle, shown here as 210 or 212. Fig. 3A shows the processor 112 with the generic lead 140 removed. Fig. 3B shows the processor 112 with the generic lead 140 attached. The male connector 142 is exchangeable, and acts as a seal to prevent or minimize fluid transfer into the processor 112.

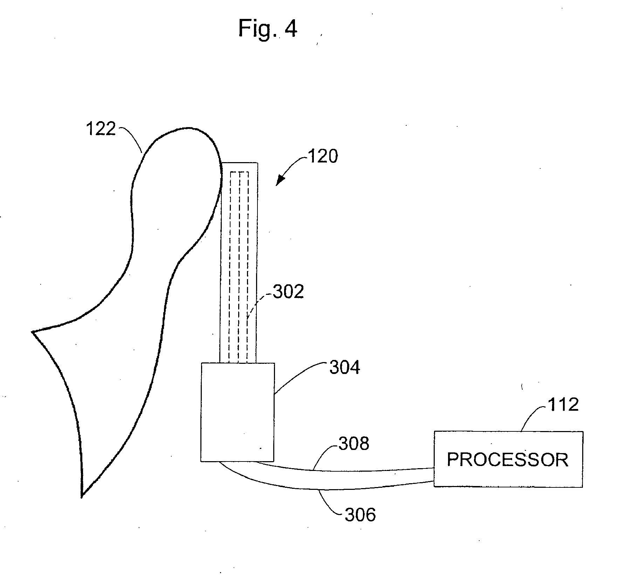

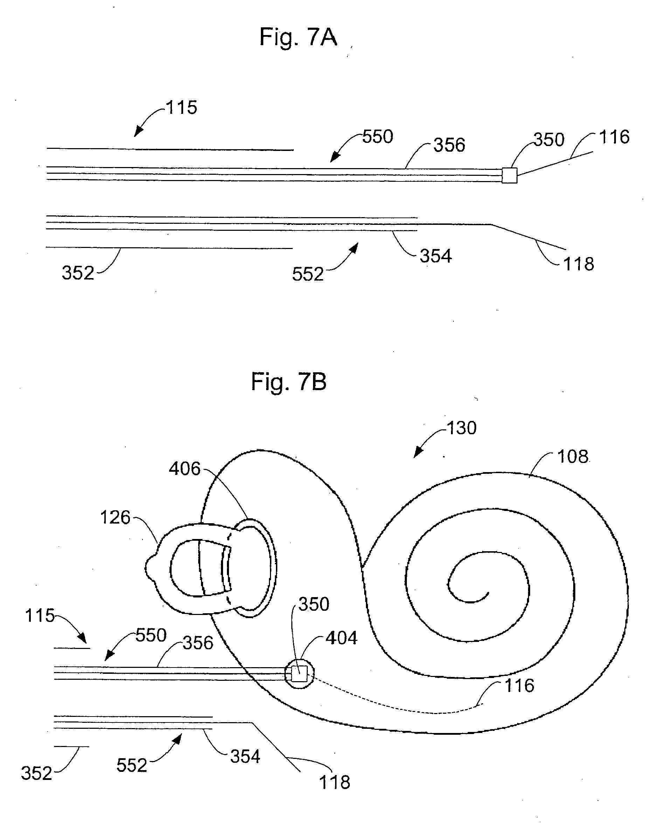

Referring to Fig. 4, an embodiment of the sensor 120 of a fully-implantable cochlear implant in accordance with the present invention is shown. Here, the sensor 120 is touching the malleus 122. The sensor may be composed of at least a cantilever 302 within a sensor housing 304. The sensor 120 may be connected to the processor 112 by at least two wires 306 and 308, which may form first lead 114. Both wires are preferably made of biocompatible materials, but not necessarily the same biocompatible material. Examples of such biocompatible materials would be tungsten, platinum, palladium, and the like. The wires 306 and 308 may or may not be coated with a coating (see Fig. 7 and the accompanying description for an example of a coating). One, both, or neither of the wires 306 and 308 may be disposed inside a casing (see Fig. 7 and the accompanying description for an example of a casing). The cantilever 302 should have at least two ends, where at least one end is in operative contact with the tympanic membrane or one or more bones of the ossicular chain. The cantilever 302 may be a laminate of at least two layers of material. The material used may be piezoelectric. One example of such a cantilever 302 is a piezoelectric bimorph, which is well-known in the art (see for example, U.S. 5,762,583). In one embodiment, the cantilever is made of two layers of piezoelectric material. In another embodiment, the cantilever is made of more than two layers of piezoelectric material. In yet another embodiment, the cantilever is made of more than two layers of piezoelectric material and non-piezoelectric material.

The sensor housing 304 of the sensor 120 may be made of a biocompatible material. In one embodiment, the biocompatible material may be titanium or gold. In another embodiment, the sensor 120 may be similar to the sensor described in U.S. 7,524,278 to Madsen et al., or available sensors, such as that used in the ESTEEM™ implant (Envoy Medical, Corp., St. Paul, MN), for example.

In alternative embodiments, the sensor 120 may be an electromagnetic sensor, an optical sensor, or an accelerometer. Accelerometers are known in the art, for example, as described in US 5,540,095.

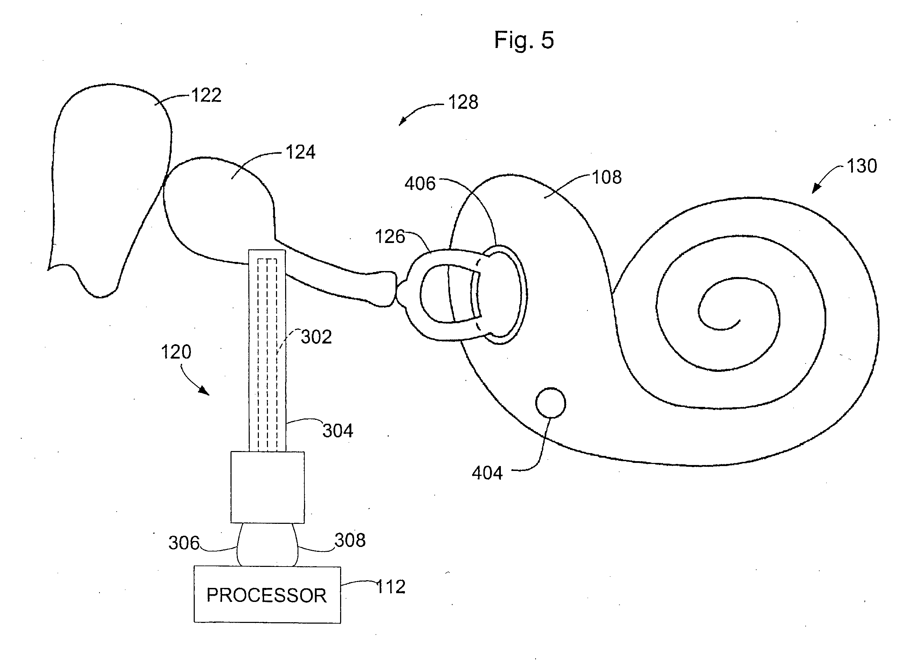

Referring to Fig. 5, an embodiment of the sensor 120 of a fully-implantable cochlear implant in accordance with the present invention is shown. Also shown are portions of the subject's anatomy, which includes, if the subject is anatomically normal, at least the malleus 122, incus 124, and stapes 126 of the middle ear 128, and the cochlea 108, oval window 406, and round window 404 of the inner ear 130. Here, the sensor 120 is touching the incus 124. The sensor 120 in this embodiment can be as described for the embodiment shown in Fig. 4. Further, although not shown in a drawing, the invention contemplates a sensor 120 that instead may be in operative contact with the tympanic membrane or the stapes, or a combination of the tympanic membrane 106, malleus 122, incus 124, or stapes 126.

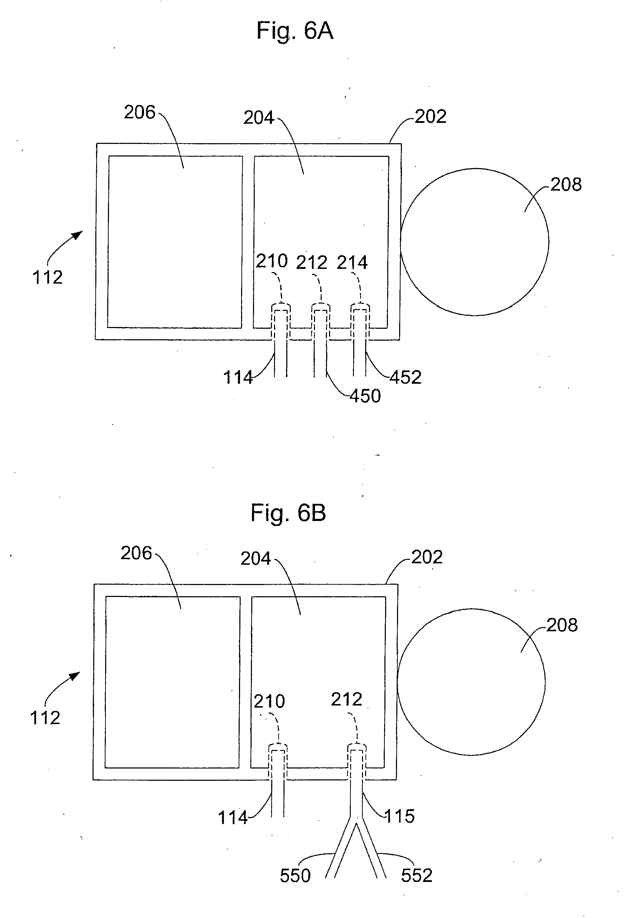

Referring to Figs. 6A-C, two embodiments of the processor portion 112 of a fully-implantable cochlear implant in accordance with the present invention are shown. The processor 112 is itself composed of at least a housing 202, an electronics unit 204, a rechargeable battery 206, and a coil 208. The housing 202 can be constructed of any biocompatible material. Preferably the biocompatible material is titanium. Also, preferably, the housing 202 is hermetically sealed.

Fig. 6A shows an embodiment in accordance with the present invention showing three female receptacles, first female receptacle 210, second female receptacle 212, and third female receptacle 214, for insertion of the first, second, and third leads, 114, 450, and 452, respectively.

Fig. 6B shows an embodiment in accordance with the present invention showing two female receptacles, first female receptacle 210 and second female receptacle 212, for insertion of first lead 114 and combination lead 115 respectively. Combination lead 115 splits in a location distal from the point of attachment to the processor 112 into at least two leads, here labeled first conductor 550 and second conductor 552. The relationships between the portions of the processor and the leads or conductors are not meant to be limited to what is shown in the drawings. For example, the sizes, placements, and arrangement of leads may be different than illustrated, as may be the sizes, placements, and arrangements of the various portions of the processor.

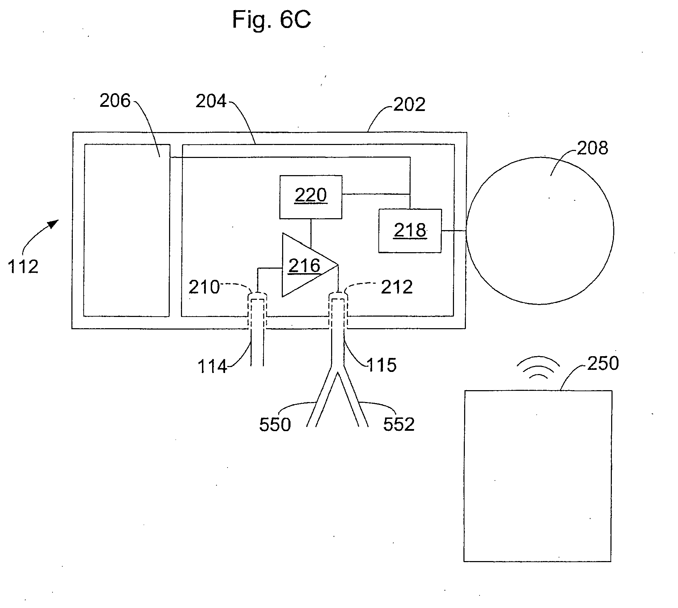

Fig. 6C is a more detailed schematic of the electronics unit 204 shown in Fig. 6B. A telemetry and power management (TPM) circuit 218 is provided. The telemetry and power management circuit 218 operates in two modes. In one mode, it receives power from the coil 208, which has received it transcutaneosly from the external device or transmission coil 250. The TPM circuit 218 then delivers the power to the battery, which may be such as rechargeable battery 206, to recharge it.

In another mode of operation, the coil 208 receives telemetry from the external device or transmission coil 250, and can deliver controller information to the TPM circuit 218. The TPM circuit 218 then delivers the controller information to the controller 220. The controller 220 can then communicate the controller information to the amplifier 216 to make adjustments in the operation of the electronics unit 204 (e.g., make it louder, make it softer, etc.).

Any device which can transmit power and telemetry can be used as the external device or coil 250. Such devices are known in the art. It is well within the knowledge of one of ordinary skill in the art, given the present disclosure, to design and program the electronics unit of the present invention.

The signal from the sensor 120 (Fig. 2) enters the electronics unit 204 via the first lead 114 and the first female receptacle 210. The sensor signal passes into the amplifier 216 and is output to the second female receptacle 212 and combination lead 115 to be delivered to the inner ear and interpreted as sound. The electronics unit 204 may be any suitable electronics unit that can interpret and manipulate the electrical signals from the sensor and generate an output electrical signal to the cochlea via a cochlear electrode, where the output electrical signals are ultimately interpreted by the subject as auditory information.

The system according to the present invention includes a power source. The power source provides power for the processor means, electrode array and any other electrical or electronic componentry of the implant system.

The power source can be a battery. The battery is preferably rechargeable. The battery should have a high charge density and be rechargeable over a considerable number of charge/discharge cycles.

The rechargeable battery 206 can be, but does not have to be, any rechargeable battery known in the hearing aid, hearing implant; or medical implant arts.

Preferably the rechargeable battery 206 is a lithium-ion battery. Also preferably, the rechargeable battery 206 is rechargeable by telemetry. The rechargeable battery 206 provides power to electronics unit 204 through connections standard in the art, such as those found in the in the ESTEEM™ implant (Envoy Medical Corp., St. Paul, MN), although any available suitable method of connecting the rechargeable battery 206 to the electronics unit 204 may be used without exceeding the scope of this disclosure.

Referring to Fig. 7A, an embodiment of the distal end of combination lead 115 of a fully-implantable cochlear implant device in accordance with the present invention is shown. Combination lead 115 is attached to the processor 112 at one end (Fig. 6B) and extends into the ear of the subject, an example of which is shown in Fig. 7B.

Fig. 7A shows a close-up schematic of the distal end of combination lead 115. First conductor 550 and second conductor 552 are disposed within a sheath or casing 352, which may be insulated. First conductor 550 is composed of a cochlear electrode 116 coated in a coating 356. The coating 356 of first conductor 550 ends at a point along the cochlear electrode 116 distal to the site of attachment 212 to the processor 112 (Fig. 6B). The point at which the coating 356 ends leaves a sufficient amount of cochlear electrode 116 uncoated so that the cochlear electrode 116 can be properly inserted into the subject's tissue enabling the cochlear electrode 116 to achieve its intended function.

Also included in first conductor 550 is a radioactive marker 350. The radioactive marker 350 may be part of the coating 356, or it may be separate. It may be made with of the same material as coating 356, or it may be made with a different material. The radioactive marker may be placed at the point on the cochlear electrode 116 where the coating 356 ends, or it may be placed at another point along the cochlear electrode 116 or at another point along first conductor 550.

Alternatively, the radioactive marker may be part of the cochlear electrode 116 itself. The invention contemplates using any known radioactive isotope as the marker, but preferably a radioactive isotope known and used in the medical arts, and more preferably barium sulfate or tungsten. In one preferable embodiment, the invention contemplates using a radioactive imbued plastic, such as those offered commercially as RADIOPAQUE™ (RTP Company, Winona, MN).

Second conductor 552 is composed of a ground electrode 118 coated in a second coating 354, which may be the same as coating 356. The second coating 354 of second conductor 552 ends at a point along the ground electrode 118 distal to the site of attachment to the processor 112 (Fig. 6B). The point at which the coating 354 ends leaves a sufficient amount of ground electrode 118 uncoated so that the ground electrode 118 can be properly inserted into the subject's tissue enabling the ground electrode 118 to achieve its intended function.

Fig. 7B shows an embodiment of the distal end of combination lead 115 of a fully-implantable cochlear implant device in accordance with the present invention. Combination lead 115 is attached to the processor 112 at one end (Fig. 6B) and extends into the ear of the subject, which if anatomically normal, includes at least a stapes 126, an oval window 406, a round window 404, and a cochlea 108. First conductor 550 is placed such that the uncoated cochlear electrode 116 extends through the round window 404 into the cochlea 108.

Shown in Fig. 7B is an embodiment where the cochlear electrode 116 is a short electrode that does not make numerous turns around the conch shape of the cochlea 108. In other embodiments, the cochlear electrode 116 may be longer and extend further into the cochlea 108. In still other embodiments, the cochlear electrode 116 may be long enough to navigate a turn or turns in the conch shape of the cochlea 108.

In a preferred embodiment, the marker 350 is shown in Fig. 7B as being placed just outside the round window. However, the marker 350, as discussed above, can be placed at any point along the first conductor 550 or cochlear electrode 116, as long as the radioactive marker 350 maintains its function of being able to provide information about the placement of the cochlear electrode 116.

Also shown in Fig. 7B is second conductor 552 and ground electrode 118. The ground electrode 118 is placed in the subject's tissue outside the inner ear 130.

The casing 352 is an electrically insulating material. The coatings 356 and 354 on first conductor 550 and second conductor 552, respectively, are also electrically insulating materials. The electrically insulating materials used may be the same in all instances, or may be different in all instances, or any combination. In one embodiment, the electrically isolating material is a biocompatible silicone. In alternative embodiments, the electrically isolating material is a biocompatible polyurethane. Similar casings and coatings may be used for any other leads, wires, or electrodes of the invention, for example, leads 114 and 115 (shown at least in Fig. 6B).

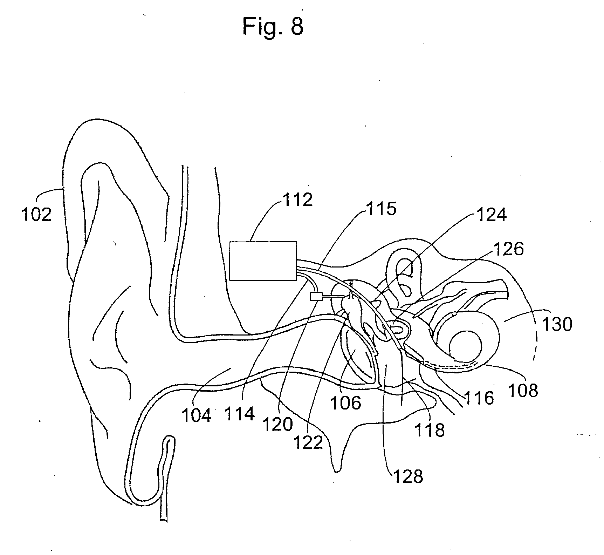

Referring to Fig. 8, an embodiment of a fully-implantable cochlear implant device in accordance with the present invention is shown in the hearing impaired subject's head, who if anatomically normal, possesses a pinna 102, an ear canal 104, a tympanic membrane 106, an ossicular chain composed of a malleus 122, an incus 124, and a stapes 126 disposed within a middle ear space 128, and an inner ear 130 including a cochlea 108. The device in this embodiment is composed of: a processor 112; a first lead 114 connecting the sensor 120 to the processor 112; the sensor 120, shown here touching the malleus 122; and a combination lead 115 attached to the processor 112. Combination lead 115 contains both a ground electrode 118 and a cochlear electrode 116. In this embodiment, unlike the embodiment shown in Fig. 1, the cochlear electrode 116 is a short electrode that does not make numerous turns around the conch shape of the cochlea 108.

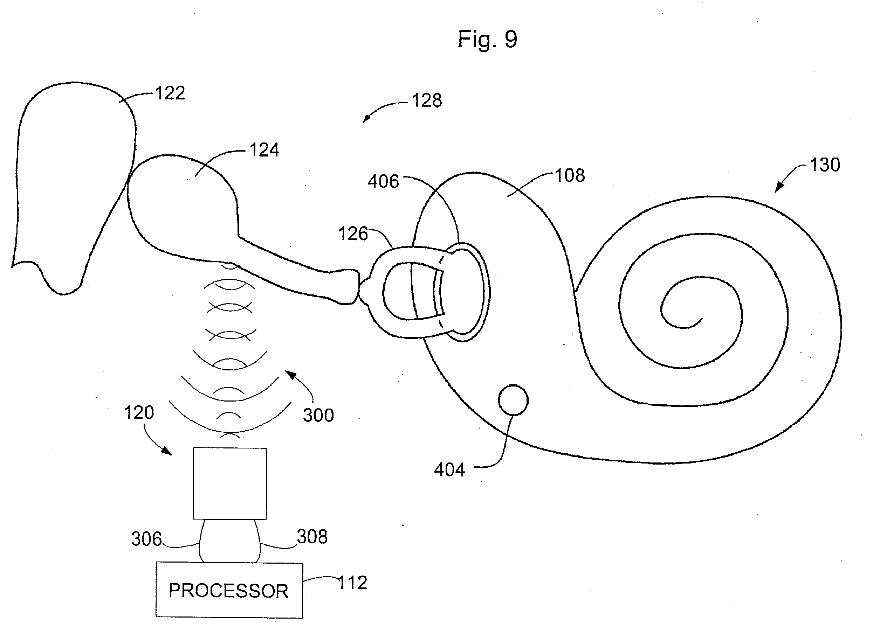

FIG. 9 illustrates an embodiment having electronics or processor 112, previously discussed. It should be understood that the electronics within the processor 112 can vary, depending on the embodiment of the invention. However, for ease of illustration, the same number (112) is used throughout to indicate the processor, even though the embodiment of the processor may vary.

The processor 112 is preferably hermetically sealed. The embodiment of FIG. 9 can have a transceiver or sensor 120 for transmitting signals to a bone in the ossicular chain, and receiving signals which have been reflected back from the ossicular bone, directly or indirectly.

Signals 300 are shown being directed at incus 124, and being reflected back from the incus 124 to the sensor 120. The signals can be sonic, ultrasonic, IR, RF, or laser signals in various embodiments. Transceiver or sensor 120 can be coupled to processor 112 via wires 306 and 308.

In some embodiments, some signal pre-processing is done remotely from processor 112, for example, near or in sensor 120. Also contemplated is a method of improving the hearing of a subject by using fully-implantable, microphoneless implants described above. The method may include the step of implanting the fully-implantable, microphoneless cochlear implant into a hearing impaired subject, thereby improving the hearing of the subject.

The indefinite articles "a" and "an," as used herein in the specification and in the claims, unless clearly indicated to the contrary, should be understood to mean "at least one." The phrase "or," as used herein in the specification and in the claims, should be understood to mean "either or both" of the elements so conjoined, i.e., elements that are conjunctively present in some cases and disjunctively present in other cases.

In accordance with the provisions of the patent statutes, the present invention has been described in what is considered to represent its preferred embodiments. However, it should be noted that the invention can be practiced otherwise than as specifically illustrated and described without departing from its spirit or scope.

The present subject-matter includes, inter alia, the following aspects:
1. A fully implantable apparatus for improving the hearing of a subject, said apparatus comprising:
   a) a means for sensing vibrations impinging upon the tympanic membrane of the subject or something in operative contact with the tympanic membrane,
   b) a means for converting said vibrations to an electrical signal, and
   c) a means for transmitting said electrical signal to the inner ear of the subject;
2. The fully implantable apparatus defined in aspect 1, wherein the means for sensing vibrations comprise:
   a) a sensor suitable for operative contact with the tympanic membrane of the subject to convert mechanical vibrations sensed by the sensor into electrical signals,
   b) a processor electrically connected to the sensor to process the electrical signals received by the processor, and produce an output electrical signal representative of the mechanical vibrations sensed by the sensor, and
   c) a cochlear electrode electrically connected to the processor and suitable for implantation in the cochlea of the subject to enable the subject to sense the output electrical signal as auditory information, and
   d) a ground electrode electrically connected to the processor to provide a return path for the output electrical signal;
3. The fully implantable apparatus defined in aspect 2, wherein the sensor comprises a cantilever and a sensor housing;
4. The fully implantable apparatus defined in any of the aspects 2 or 3, wherein the processor comprises:
   a) an electrical unit,
   b) a battery connected to the electrical unit to power the electrical unit, and
   c) a coil connected to the electrical unit;
5. The fully implantable apparatus defined in aspect 4, wherein the electrical unit further comprises:
   a) a telemetry and power management circuit electrically connected to the coil and the battery,
   b) a controller electrically connected to the telemetry and power management circuit and the battery,
   c) an amplifier electrically connected to the controller, and
   d) a first female receptacle and a second female receptacle electrically connected to the amplifier;
6. The fully implantable apparatus defined in aspect 5, wherein the telemetry and power management circuit, the controller and the amplifier are contained within a housing;
7. The fully implantable apparatus defined in aspect 6 wherein the first female receptacle and the second female receptacle are mounted to the housing;
8. The fully implantable apparatus defined in aspect 7, and further comprising an external device or transmitter coil to transcutaneously transmit power or information to the coil;
9. The fully implantable apparatus defined in any of the aspects 5 to 8, wherein the cochlear electrode is removably connected to the first female receptacle;
10. The fully implantable apparatus defined in any of the aspects 5 to 8, wherein the ground electrode is removably connected to the second female receptacle;
11. The fully implantable apparatus defined in any of the aspects 2 to 10, wherein the cochlear electrode includes a radioactive marker;
12. The fully implantable apparatus defined in any of the aspects 2 to 10, wherein the cochlear electrode comprises:
   a) a lead,
   b) a coating covering the lead,
   c) an electrical conductor extending past the coating;
13. The fully implantable apparatus defined in aspect 12, wherein a radioactive marker is provided in the coating;
14. A method for improving the hearing of a subject, said method comprising the steps of:
   a) sensing vibrations impinging upon the tympanic membrane of the subject, or something in operative contact with the tympanic membrane;
   b) converting the vibrations to an electrical signal, and
   c) transmitting the electrical signal to the inner ear of the subject as an electrical signal or stimulus that will be interpreted as auditory information;
15. The method defined in aspect 14, wherein the vibrations are sensed by a cantilever in contact with the tympanic membrane of the subject;
16. The method defined in aspect 15, wherein the vibrations sensed by the cantilever are converted to an electrical signal by a processor;
17. The method defined in aspect 16, wherein the electrical signal from the processor is transmitted to the inner ear by a cochlear electrode implanted in the cochlea of the subject;
18. The method defined in any of the aspects 15 to 17, wherein the cantilever is in contact with the stapes;
19. The method defined in any of the aspects 15 to 17, wherein the cantilever is in contact with the incus;
20. The method defined in any of the aspects 15 to 17, wherein the cantilever is in contact with the malleus;
21. The fully implantable hearing apparatus defined in any of the aspects 2 to 13, wherein the sensor is connected to the processor by at least two wires;
22. The fully implantable hearing apparatus defined in aspect 21, wherein at least two wires may be made of biocompatible materials;
23. The fully implantable hearing apparatus defined in aspect 22, wherein both wires are not made of the same biocompatible materials;
24. The fully implantable hearing apparatus defined in aspect 23, wherein the biocompatible materials are chosen from the group consisting of tungsten, platinum, iridium, palladium, and the like, as well as alloys thereof;
25. The fully implantable hearing apparatus defined in aspect 3, wherein the cantilever is composed of a laminate of at least two layers of material;
26. The fully implantable hearing apparatus defined in aspect 25, wherein at least one of the at least two layers of material is piezoelectric;
27. The fully implantable hearing apparatus defined in aspect 3, wherein the cantilever is a piezoelectric bimorph;
28. The fully implantable hearing apparatus defined in aspect 3, wherein the cantilever is made of two layers of piezoelectric material;
29. The fully implantable hearing apparatus defined in aspect 3, wherein the cantilever is made of more than two layers of piezoelectric material and non-piezoeiectric material;
30. The fully implantable hearing apparatus defined in any of the aspects 3 or 25 to 29, wherein the sensor housing is made of a biocompatible material;
31. The fully implantable hearing apparatus defined in aspect 30, wherein the biocompatible material is titanium;
32. The fully implantable hearing apparatus defined in aspect 30, wherein the biocompatible material is gold;
33. The fully implantable hearing apparatus defined in any of the aspects 3 or 25 to 32, wherein the sensor is an electromagnetic sensor;
34. The fully implantable hearing apparatus defined in any of the aspects 3 or 25 to 32, wherein the sensor is an optical sensor;
35. The fully implantable hearing apparatus defined in any of the aspects 3 or 25 to 32, wherein the sensor is an accelerometer;
36. The fully implantable hearing apparatus defined in any of the aspects 4 to 10, wherein the battery is rechargable battery;
37. The fully implantable hearing apparatus defined in aspect 36, wherein the rechargable battery is a lithium ion battery;
38. The fully implantable hearing apparatus defined in any of the aspects 11 or 13, wherein the radioactive marker is a radioactive isotope;
39. The fully implantable hearing apparatus defined in aspect 38, wherein the radioactive isotope is barium sulfate;
40. The fully implantable hearing apparatus defined in aspect 38, wherein the radioactive isotope is tungsten;
41. The fully implantable hearing apparatus defined in any of the aspects 11 or 13, wherein the radioactive marker is a radioactive imbued plastic;
42. The fully implantable hearing apparatus defined in aspect 12, wherein the coating covering the lead is an electrically insulating material;
43. The fully implantable hearing apparatus defined in aspect 42, wherein the electrically insulating material is a biocompatible silicone;
44. The fully implantable hearing apparatus defined in aspect 42, wherein the electrically insulating material is biocompatible polyurethane;
45. An implantable apparatus for improving the hearing of a subject, said apparatus comprising:
   a) a processor to process input electrical signals received by the processor through a female receptacle and produce an output electrical signal representative of the input electrical signal,
   b) a cochlear electrode electrically connected to the processor and suitable for implantation in the cochlea of a person to enable the person to sense the output electrical signal as auditory information, and
   c) a ground electrode electrically connected to the processor to provide a return path for thee output electrical signal;
46. The implantable apparatus defined in aspect 45, wherein the input electrical signal is received from a separate sensor that attaches to the female receptacle.

## Claims

1. A fully implantable apparatus for improving the hearing of a subject, said apparatus comprising:
a) a means for sensing vibrations impinging upon the tympanic membrane of the subject or something in operative contact with the tympanic membrane,
b) a means for converting said vibrations to an electrical signal, and
c) a means for transmitting said electrical signal to the inner ear of the subject.

2. The fully implantable apparatus defined in claim 1, wherein the means for sensing vibrations comprise:
a) a sensor suitable for operative contact with the tympanic membrane of the subject to convert mechanical vibrations sensed by the sensor into electrical signals,
b) a processor electrically connected to the sensor to process the electrical signals received by the processor, and produce an output electrical signal representative of the mechanical vibrations sensed by the sensor, and
c) a cochlear electrode electrically connected to the processor and suitable for implantation in the cochlea of the subject to enable the subject to sense the output electrical signal as auditory information, and
d) a ground electrode electrically connected to the processor to provide a return path for the output electrical signal.

3. The fully implantable apparatus defined in claim 2, wherein the processor comprises:
a) an electrical unit,
b) a battery connected to the electrical unit to power the electrical unit, and
c) a coil connected to the electrical unit,
wherein the electrical unit further comprises:
a) a telemetry and power management circuit electrically connected to the coil and the battery,
b) a controller electrically connected to the telemetry and power management circuit and the battery,
c) an amplifier electrically connected to the controller, and
d) a first female receptacle and a second female receptacle electrically connected to the amplifier.

4. The fully implantable apparatus defined in claim 3 wherein the first female receptacle and the second female receptacle are mounted to a housing.

5. The fully implantable apparatus defined in any of the claims 3 or 4, wherein the cochlear electrode is removably connected to the first female receptacle or to the second female receptacle.

6. The fully implantable apparatus defined in any of the claims 2 to 5, wherein the cochlear electrode includes a radioactive marker.

7. A method for improving the hearing of a subject, said method comprising the steps of:
a) sensing vibrations impinging upon the tympanic membrane of the subject, or something in operative contact with the tympanic membrane.
b) converting the vibrations to an electrical signal, and
c) transmitting the electrical signal to the inner ear of the subject as an electrical signal or stimulus that will be interpreted as auditory information.

8. The method defined in claim 7, wherein the vibrations are sensed by a cantilever in contact with the tympanic membrane of the subject, wherein the vibrations sensed by the cantilever are converted to an electrical signal by a processor, wherein the electrical signal from the processor is transmitted to the inner ear by a cochlear electrode implanted in the cochlea of the subject.

9. The method defined in any of the claims 7 or 8, wherein the cantilever is in contact with the stapes or with the incus or with the malleus.

10. The fully implantable hearing apparatus defined in any of the claims 2 to 6, wherein the sensor is connected to the processor by at least two wires, at least one of said at least two wires being made of a biocompatible material chosen from the group consisting of tungsten, platinum, iridium, and the like, as well as alloys thereof.

11. The fully implantable hearing apparatus defined in any of the claims 2 to 10, wherein the sensor comprises a cantilever and a sensor housing, wherein the cantilever is composed of a laminate of at least two layers of material.

12. The fully implantable hearing apparatus defined in claim 11, wherein at least one of the at least two layers of material is piezoelectric.

13. The fully implantable hearing apparatus defined in any of the claims 2 to 10, wherein the sensor comprises a cantilever and a sensor housing, wherein the cantilever is made of two or more layers of material, wherein at least one of said two or more layers is made of a piezoelectric material.

14. The fully implantable hearing apparatus defined in any of the claims 2 to 13, wherein the sensor comprises a cantilever and a sensor housing, wherein the sensor housing is made of a biocompatible material, wherein the biocompatible material is titanium or gold.

15. The fully implantable hearing apparatus defined in any of the claims 2 to 14, wherein the sensor is an electromagnetic sensor or an optical sensor or an accelerometer.

16. The fully implantable hearing apparatus defined in claim 6, wherein the radioactive marker is a radioactive imbued plastic.

17. An implantable apparatus for improving the hearing of a subject, said apparatus comprising:
a) a processor to process input electrical signals received by the processor through a female receptacle and produce an output electrical signal representative of the input electrical signal,
b) a cochlear electrode electrically connected to the processor and suitable for implantation in the cochlea of a person to enable the person to sense the output electrical signal as auditory information, and
c) a ground electrode electrically connected to the processor to provide a return path for thee output electrical signal.

18. The implantable apparatus defined in claim 17, wherein the input electrical signal is received from a separate sensor that attaches to the female receptacle.
